# EUROPEAN PATENT APPLICATION

(11) **EP 4 318 320 A1**
(43) Date of publication of application: **07.02.2024**
(21) Application number: 22780299.8
(22) Date of filing: 22.03.2022
(51) Int. Cl.: G06N 3/08, G06N 20/00, A61B 5/16, G06F 3/01

(54) **MODEL GENERATION METHOD, COMPUTER PROGRAM, INFORMATION PROCESSING DEVICE, INFORMATION PROCESSING SYSTEM, INFORMATION PROCESSING METHOD, AND METHOD FOR GENERATING TRAINING DATA**

(30) Priority: 31.03.2021 JP 2021061231
(71) Applicant: Mitsui Chemicals, Inc., Tokyo 104-0028 (JP)
(72) Inventor: YOSHIDA, Mitsunobu, Nagoya-shi, Aichi 457-0801 (JP); MARUKO, Nobuhiro, Nagoya-shi, Aichi 457-0801 (JP); KAWAHARA, Iori, Tokyo 107-0061 (JP); KAWAHARA, Tatsuo, Tokyo 107-0061 (JP)
(74) Representative: Hoffmann Eitle
(86) International application number: PCT/JP2022/013111
(87) International publication number: WO 2022/210084

(57) **Abstract**

A model generating method, a computer program, an information processing device, an information processing system, an information processing method, and a training data generating method are provided.

A model generating method causing a computer to execute processing of acquiring electroencephalographic data of subjects based on outputs of an electroencephalograph, acquiring sensitivity information of the subjects by inputting the acquired electroencephalographic data to a first learning model trained to output the sensitivity information in accordance with input of the electroencephalographic data, acquiring biological data of the subjects based on outputs of a piezoelectric element, and generating a second learning model for outputting the sensitivity information of a person being tested by using a data set including the acquired biological data and the sensitivity information of the subjects acquired from the first learning model as training data when the biological data of the person measured by the piezoelectric element is input.

## Description

### [Technical Field]

The present invention relates to a model generating method, a computer program, an information processing device, an information processing system, an information processing method, and a training data generating method.

### [Background Art]

Recently, it has been considered that a piezoelectric element containing a helically chiral polymer is applied to a piezoelectric device such as a sensor and an actuator (for example, refer to Patent Documents 1 to 4).

### [Prior Art Document]

### [Patent Document]

[Patent Document 1] Japanese Patent No. 4934235
[Patent Document 2] International Publication No. 2010/104196
[Patent Document 3] Japanese Patent Laid-Open Publication No. H10-132669
[Patent Document 4] International Publication No. 2014/058077

### [Summary of Invention]

### [Problems to be Solved by Invention]

However, currently, there is no technology of outputting sensitivity information of a user on the basis of the output of the piezoelectric element described above.

An object of the present invention is to provide a model generating method, a computer program, an information processing device, an information processing system, an information processing method, and a training data generating method in which sensitivity information of a user is obtained from the output of a piezoelectric element.

### [Means for Solving Problems]

A model generating method according to an aspect of the invention, causes a computer to execute processing of acquiring electroencephalographic data of subjects based on outputs of an electroencephalograph, acquiring sensitivity information of the subjects by inputting the acquired electroencephalographic data to a first learning model trained to output the sensitivity information in accordance with input of the electroencephalographic data, acquiring biological data of the subjects based on outputs of a piezoelectric element, and generating a second learning model for outputting the sensitivity information of a person being tested by using a data set including the acquired biological data and the sensitivity information of the subjects acquired from the first learning model as training data when the biological data of the person measured by the piezoelectric element is input.

The model generating method according to an aspect of the invention, wherein the training data further includes sensor data obtained by other sensors, and when the biological data and the sensor data are input, the second learning model is trained to output the sensitivity information.

The model generating method according to an aspect of the invention, wherein the biological data is at least one time-series data piece of a respiration rate, a heart rate, and a body movement value of the subjects.

The model generating method according to an aspect of the invention, wherein the sensor data is at least one time-series data piece of an environmental temperature, and a body temperature and a blood-pressure value of the subjects.

The model generating method according to an aspect of the invention, wherein the sensitivity information includes at least one of calmness, sleepiness, concentration, preference, interest, stress, irritation, comfort, satisfaction, and dissatisfaction of the subjects.

The model generating method according to an aspect of the invention, wherein the piezoelectric element is a line-shaped or film-shaped piezoelectric element containing a non-pyroelectric organic piezoelectric material.

The model generating method according to an aspect of the invention, wherein the biological data is acquired from the piezoelectric element arranged in a state of not being in contact with skins of the subjects while acquiring the electroencephalographic data from the electroencephalograph mounted on heads of the subjects.

A computer program according to an aspect of the invention, causes a computer to execute processing of acquiring biological data of a user based on an output of a non-pyroelectric piezoelectric element arranged in a state of not being in contact with a skin of the user, acquiring sensitivity information of the user by inputting the acquired biological data of the user to a learning model trained to output the sensitivity information in accordance with input of the biological data, and outputting information based on the acquired sensitivity information.

The computer program according to an aspect of the invention, wherein the learning model is trained by using a data set that includes sensitivity information of subjects estimated by using another learning model based on electroencephalographic data of the subjects and includes biological data of the subjects based on outputs of the piezoelectric element as training data.

The computer program according to an aspect of the invention, wherein the learning model is trained by using the data set further including sensor data obtained by other sensors as the training data.

The computer program according to an aspect of the invention, wherein the biological data is at least one time-series data piece of a respiration rate, a heart rate, and a body movement value of the user.

The computer program according to an aspect of the invention, wherein the sensor data is at least one time-series data piece of an environmental temperature, and a body temperature and a blood-pressure value of the user.

The computer program according to an aspect of the invention, causes a computer to execute processing of acquiring attribute information of the user, selecting one learning model from a plurality of learning models prepared in accordance with attribute of the user, based on the acquired attribute information of the user, and acquiring the sensitivity information of the user by inputting the biological data of the user to the selected one learning model.

The computer program according to an aspect of the invention, causes a computer to execute processing of receiving correction of the sensitivity information from the user, and relearning the learning model by using training data including the corrected sensitivity information and the biological data measured for the user as a data set.

The computer program according to an aspect of the invention, causes a computer to execute processing of acquiring first sensitivity information by inputting first biological data of the user measured by the piezoelectric element in a first environment to the learning model, acquiring second sensitivity information by inputting second biological data of the user measured by the piezoelectric element in a second environment to the learning model, and outputting information of the first environment and the first sensitivity information acquired from the learning model in association with each other, and outputting information of the second environment and the second sensitivity information acquired from the learning model in association with each other.

The computer program according to an aspect of the invention, causes a computer to execute processing of acquiring first sensitivity information by inputting first biological data of the user measured by the piezoelectric element in a situation in which a first offering is provided to the user to the learning model, acquiring second sensitivity information by inputting second biological data of the user measured by the piezoelectric element in a situation in which a second offering is provided to the user to the learning model, and outputting information of the first offering and the first sensitivity information acquired from the learning model in association with each other, and outputting information of the second offering and the second sensitivity information acquired from the learning model in association with each other.

An information processing device according to an aspect of the invention, includes a biological data acquisition unit acquiring biological data of a user based on an output of a non-pyroelectric piezoelectric element arranged in a state of not being in contact with a skin of the user, a sensitivity information acquisition unit acquiring sensitivity information of the user by inputting the acquired biological data of the user to a learning model trained to output the sensitivity information in accordance with input of the biological data, and an output unit outputting information based on the acquired sensitivity information.

An information processing system according to an aspect of the invention, includes a non-pyroelectric piezoelectric element arranged in a state of not being in contact with a skin of a user, and an information processing device that includes, a biological data acquisition unit acquiring biological data of the user based on an output of the piezoelectric element, a sensitivity information acquisition unit acquiring sensitivity information of the user by inputting the acquired biological data of the user to a learning model trained to output the sensitivity information in accordance with input of the biological data, and an output unit outputting information based on the acquired sensitivity information.

An information processing method according to an aspect of the invention, causes a computer to execute processing of acquiring biological data of a user based on an output of a non-pyroelectric piezoelectric element arranged in a state of not being in contact with a skin of the user, acquiring sensitivity information of the user by inputting the acquired biological data of the user to a learning model trained to output the sensitivity information in accordance with input of the biological data, and outputting information based on the acquired sensitivity information.

A training data generating method according to an aspect of the invention, causes a computer to execute processing of acquiring electroencephalographic data of subjects based on an output of an electroencephalograph, acquiring sensitivity information of the subjects by inputting the acquired electroencephalographic data to a first learning model trained to output the sensitivity information in accordance with input of the electroencephalographic data, acquiring biological data of the subjects based on output of the non-pyroelectric piezoelectric element arranged in a state of not being in contact with a skin of the subjects, and generating a data set including the acquired sensitivity information and the acquired biological data as training data used for learning a second learning model trained to output the sensitivity information of the user when the biological data of the user is input.

### [Effects of Invention]

According to the present application, it is possible to acquire the sensitivity information of the user from the output of the piezoelectric element.

### [Brief Description of Drawings]

FIG. 1 is a block diagram describing a configuration of an information processing device according to Embodiment 1.
FIG. 2 is a schematic view illustrating a mounting example of a piezoelectric element.
FIG. 3 is a schematic view illustrating a configuration example of a biological information estimation model.
FIG. 4 is a schematic view illustrating a configuration example of a sensitivity information estimation model.
FIG. 5 is a schematic view illustrating a modification example of the sensitivity information estimation model.
FIG. 6 is a flowchart describing a procedure of processing executed by the information processing device during estimation.
FIG. 7 is a schematic view illustrating a display example of sensitivity information.
FIG. 8 is a block diagram describing a configuration of a learning device according to Embodiment 2.
FIG. 9 is a schematic view illustrating a configuration example of an electroencephalographic model.
FIG. 10 is a flowchart describing a generating procedure of training data.
FIG. 11 is a schematic view illustrating an example of a confirmation window.
FIG. 12 is a flowchart illustrating a relearning procedure executed by a learning device.
FIG. 13 is a flowchart describing a procedure of processing executed by an information processing device according to Embodiment 4.
FIG. 14 is a schematic view illustrating an output example in Embodiment 4.
FIG. 15 is a flowchart describing a procedure of processing executed by an information processing device according to Embodiment 5.
FIG. 16 is a schematic view illustrating an output example in Embodiment 5.
FIG. 17 is an explanatory diagram describing a learning model prepared in Embodiment 6.
FIG. 18 is a flowchart describing a procedure of processing executed by an information processing device according to Embodiment 6.

### [Mode for Carrying out Invention]

Hereinafter, the present invention will be described in detail on the basis of the drawings illustrating embodiments thereof.

### (Embodiment 1)

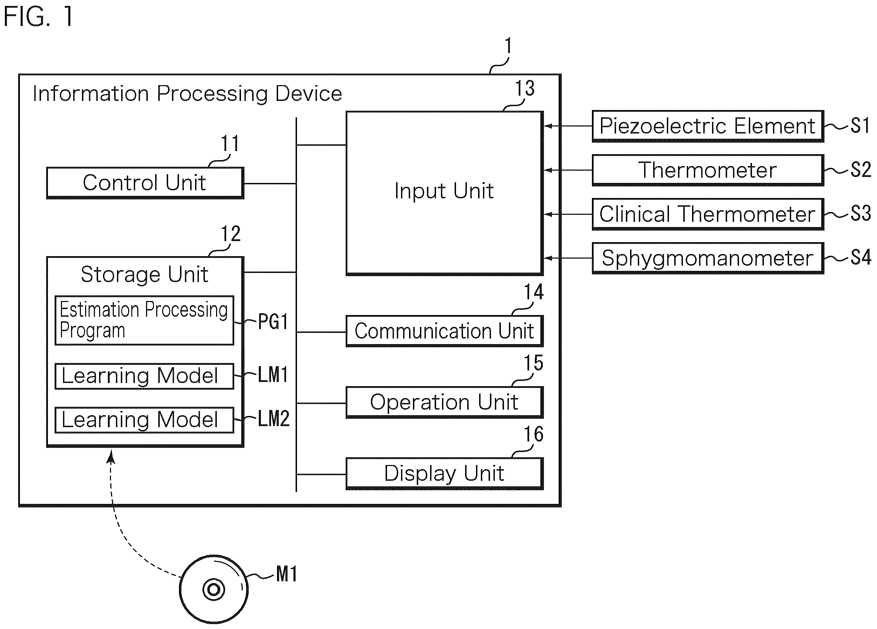

FIG. 1 is a block diagram illustrating the configuration of an information processing device according to Embodiment 1. An information processing device 1 is a dedicated or generalized computer, and includes a control unit 11, a storage unit 12, an input unit 13, a communication unit 14, an operation unit 15, a display unit 16, and the like. The information processing device 1 acquires biological data of a user on the basis of the output of a piezoelectric element S1, and acquires sensitivity information of the user on the basis of the acquired biological data. The information processing device 1 outputs information based on the acquired sensitivity information, for example, to the display unit 16.

The control unit 11, for example, includes a central processing unit (CPU), a read only memory (ROM), a random access memory (RAM), and the like. In the ROM of the control unit 11, a control program for controlling the operation of each hardware unit of the information processing device 1, and the like are stored. The CPU in the control unit 11 reads in and executes the control program stored in the ROM or various computer programs stored in the storage unit 12, and controls the operation of each hardware unit, and thus, allows the entire device to function as an information processing device according to the present invention. In the RAM of the control unit 11, data that is used during the execution of an arithmetic operation is temporarily stored.

In the embodiment, the control unit 11 includes the CPU, the ROM, and the RAM, and may be one or a plurality of arithmetic circuits including a graphics processing unit (GPU), a field programmable gate array (FPGA), a digital signal processor (DSP), a quantum processor, a volatile or non-volatile memory, and the like. In addition, the control unit 11 may have a function such as a clock outputting date and time information, a timer measuring an elapsed time from when a measurement start instruction is given to when a measurement end instruction is given, and a counter counting the number.

The storage unit 12 includes a storage such as a hard disk drive (HDD), a solid state drive (SSD), and an electronically erasable programmable read only memory (EEPROM). In the storage unit 12, various computer programs executed by the control unit 11 or various data pieces used by the control unit 11 are stored.

The computer program stored in the storage unit 12 includes an estimation processing program PG1 for causing the computer to execute processing of acquiring the biological data of the user on the basis of the output of the piezoelectric element S1, acquiring the sensitivity information of the user on the basis of the acquired biological data, and outputting information based on the acquired sensitivity information. The estimation processing program PG1 may be a single computer program, or may be configured by a plurality of computer programs. In addition, the existing library may be partially used in the estimation processing program PG1.

The computer program such as the estimation processing program PG1 stored in the storage unit 12 is provided to a non-temporary recording medium M1 in which the computer program is recorded to be readable. The recording medium M1, for example, is a portable memory such as a CD-ROM, a USB memory, a secure digital (SD) card, a micro SD card, and a compact flash (Registered Trademark). The control unit 11 reads out various computer programs from the recording medium M1 by using a reader that is not illustrated reader, and stores the various read computer programs in the storage unit 12. In addition, the computer program stored in the storage unit 12 may be provided by communication through the communication unit 14. In this case, the control unit 11 may acquire the computer program through the communication unit 14, and may store the acquired computer program in the storage unit 12.

In a case where a signal output from the piezoelectric element S1 is input, the storage unit 12 includes a learning model LM1 (hereinafter, also referred to as a biological information estimation model LM1) trained to output the biological data of the user, and a learning model LM2 (hereinafter, also referred to as a sensitivity information estimation model LM2) trained to output the sensitivity information of the user in a case where the biological data of the user is input. In the storage unit 12, configuration information of a layer of each of the learning models LM1 and LM2, information of nodes included in each layer, information of weighting and bias between the nodes, and the like are stored as information for defining the learning models LM1 and LM2. A specific configuration of the learning models LM1 and LM2 will be described below in detail.

The input unit 13 is an interface for connecting various sensors. The sensor connected to the input unit 13 includes the piezoelectric element S1. In the piezoelectric element S1, for example, a line-shaped piezoelectric element formed by coaxially arranging a central conductor, a piezoelectric material layer, an outer conductor, and an outer cover toward the outside from the center. Alternatively, a film-shaped piezoelectric element in which a lower electrode layer, a piezoelectric material layer, and an upper electrode layer are laminated in this order may be used. In the piezoelectric material of the piezoelectric element S1, a non-pyroelectric piezoelectric material may be used in order to reduce the influence of a temperature change. The piezoelectric element S1 may be connected to the input unit 13 in a wired manner, or may be connected to the input unit 13 in a wireless manner.

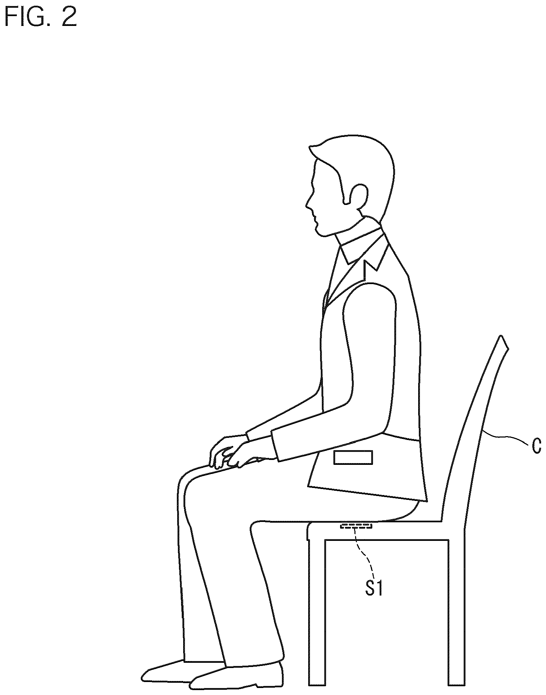

FIG. 2 is a schematic view illustrating a mounting example of the piezoelectric element S1. The piezoelectric element S1 is arranged in a state of not being in contact with the skin of the user. The piezoelectric element S1, for example, may be built in a seat portion of a chair C on which the user is seated. The piezoelectric element S1 detects a change in a load in a state where the user is seated on a time-series basis, and outputs a signal (a voltage signal) indicating a detection result to the information processing device 1. The control unit 11 of the information processing device 1 acquires time-series data (hereinafter, referred to as piezoelectric data) based on the output of the piezoelectric element S1 through the input unit 13. In this case, the voltage signal that is the output of the piezoelectric element S1 may be converted to data that is easily processed in the information processing device 1.

Note that, the piezoelectric element S1 is not limited to the seat portion of the chair C, and may be built in a backrest portion or an armrest portion. In addition, the piezoelectric element S1 may be built in a uniform or a work suit worn by the user, or may be built in a shoe insole or a bed mattress. Further, the piezoelectric element S1 may be built in a device operated by the user, such as a smart phone, a tablet, and a mouse.

At least one of a thermometer S2, a clinical thermometer S3, and a sphygmomanometer S4 may be connected to the input unit 13, in addition to the piezoelectric element S1. The thermometer S2 measures the temperature (the environmental temperature) in the vicinity of the user on a time-series basis, and outputs a signal indicating a measurement result to the information processing device 1. The clinical thermometer S3 measures the body temperature of the user on a time-series basis, and outputs a signal indicating a measurement result to the information processing device 1. The sphygmomanometer S4 measures the blood pressure of the user on a time-series basis, and outputs a signal indicating a measurement result to the information processing device 1. The control unit 11 of the information processing device 1 acquires at least one of time-series data of the environmental temperature (environmental temperature data), time-series data of the body temperature of the user (body temperature data), and time-series data of the blood pressure of the user (blood pressure data) through the input unit 13. Note that, in the following description, in a case where it is not necessary to distinguish the environmental temperature data, the body temperature data, and the blood pressure data from each other, such data pieces will also be referred to as sensor data.

Further, various sensors such as a sensor measuring the blood oxygen saturation level of the user, a sensor measuring the indoor humidity, a sensor measuring an illuminance, and a sensor measuring a noise level may be connected to the input unit 13.

The communication unit 14 includes a communication interface for transmitting and receiving various data pieces. The communication interface of the communication unit 14, for example, is a communication interface based on a communication standard of a local area network (LAN) used in WiFi (Registered Trademark) or Ethernet (Registered Trademark). In a case where data to be transmitted is input from the control unit 11, the communication unit 14 transmits the data to be transmitted to a designated destination. In addition, in a case where data transmitted from an outer device is received, the communication unit 14 outputs the received data to the control unit 11.

The operation unit 15 includes an operation device such as a touch panel, a keyboard, and a switch, and receives various operations and settings of the user. The control unit 11 performs suitable control on the basis of various operation information pieces given by the operation unit 15, and as necessary, stores setting information in the storage unit 12.

The display unit 16 includes a display device such as a liquid crystal monitor or an organic electro-luminescence (EL), and displays information to be notified to the user or the like, in accordance with an instruction from the control unit 11.

In this embodiment, the information processing device 1 includes the learning models LM1 and LM2, but the learning models LM1 and LM2 may be stored in an outer device that is accessible from the information processing device 1. In this case, the information processing device 1 may transmit data required for the arithmetic operation to the outer device such that the arithmetic operation of the learning models LM1 and LM2 is executed by the outer device, and may acquire an arithmetic operation result of the learning models LM1 and LM2 from the outer device.

Note that, the information processing device 1 is not limited to a single computer, and may be a computer system including a plurality of computers or peripherals. In addition, the information processing device 1 may be a virtual machine that is virtually constructed by software.

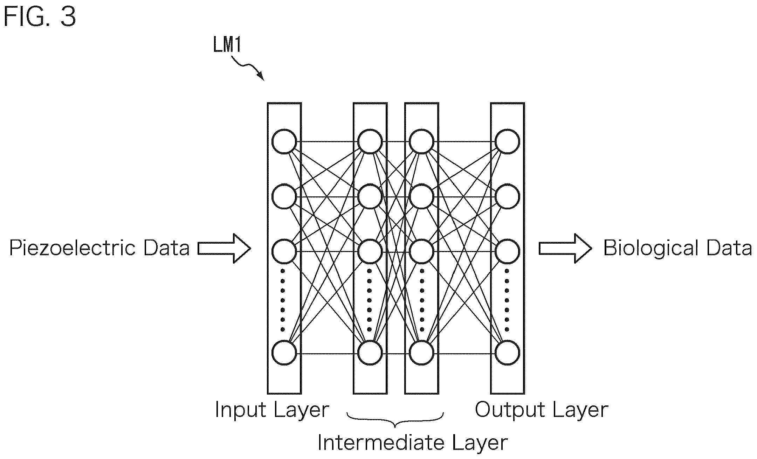

FIG. 3 is a schematic view illustrating a configuration example of the biological information estimation model LM1. In a case where the piezoelectric data based on the output of the piezoelectric element S1 is input, the biological information estimation model LM1 in Embodiment 1 is a trained learning model trained to output the biological data of the user. The piezoelectric data input to the biological information estimation model LM1 is time-series data based on the signal (the voltage signal) output from the piezoelectric element S1. In addition, the biological data output from the biological information estimation model LM1 is at least one time-series data piece of the respiration rate, the heart rate, and the body movement value of the user.

The biological information estimation model LM1 is a learning model of machine learning including deep learning, and for example, is configured by a neural network. As the neural network configuring the biological information estimation model LM1, for example, a recurrent neural network (RNN) can be used. The biological information estimation model LM1 includes an input layer, an intermediate layer (a hidden layer), and an output layer. The input layer includes T nodes to which time-series data (X₁, X₂, ..., X_{T}) with a length T is input. Here, X₁, X₂, ..., X_{T}, for example, are voltage values at each time output from the piezoelectric element S1. The intermediate layer includes a plurality of nodes for executing the arithmetic operation using set parameters (weight and bias) with respect to a numerical value given from the node of the input layer. The node of the output layer outputs a numerical value (Y_{T}) obtained by performing suitable conversion processing with respect to the numerical value given from the node of the intermediate layer. The numerical value Y_{T} output from the output layer indicates at least one value of the respiration rate, the heart rate, and the body movement value at a time T. By sequentially shifting the time-series data input to the biological information estimation model LM1, the control unit 11 is capable of acquiring at least one time-series data piece of the respiration rate, the heart rate, and the body movement value from the learning model LM1.

Note that, as a method for generating the biological information estimation model LM1, for example, methods disclosed in Japanese Patent Laid-Open Publication No. 2020-48674 and the like may be used. That is, learning is performed with a predetermined learning algorithm by using a data set including the piezoelectric data obtained by the piezoelectric element S1, and actual measurement data (ground truth data) including at least one of the respiration rate, the heart rate, and the body movement value in training data, and thus, the biological information estimation model LM1 can be generated.

The biological information estimation model LM1 in FIG. 3 includes two intermediate layers, but the number of intermediate layers is not limited to 2, and the intermediate layer can be suitably designed. The biological information estimation model LM1 may be a learning model configured by a long short-term memory (LSTM), convolutional neural networks (CNN), region-based CNN (R-CNN), and the like. The data input to the biological information estimation model LM1 may be prepared in accordance with the neural network configuring the biological information estimation model LM1. For example, in a case where the biological information estimation model LM1 is configured by the CNN or the R-CNN, image data of a graph plotted with time on a horizontal axis and the voltage value of the piezoelectric element S1 on a vertical axis may be used as the input to the input layer, instead of the time-series data.

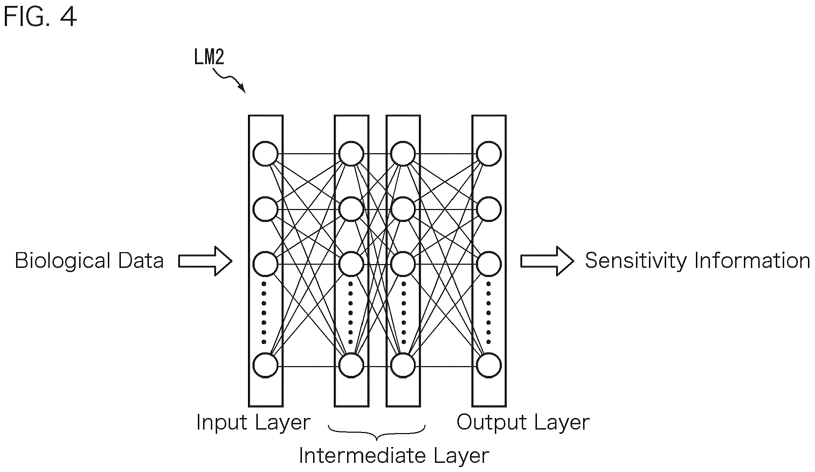

FIG. 4 is a schematic view illustrating a configuration example of the sensitivity information estimation model LM2. In a case where the biological data of the user is input, the sensitivity information estimation model LM2 in Embodiment 1 is a trained learning model trained to output the sensitivity information of the user. The biological data input to the sensitivity information estimation model LM2 is time-series biological data output from the biological information estimation model LM1. In addition, the sensitivity information output from the sensitivity information estimation model LM2 is time-series data obtained by digitizing at least one of the calmness, the sleepiness, the concentration, the preference, the interest, the stress, the irritation, the comfort, the satisfaction, and the dissatisfaction of the user.

The sensitivity information estimation model LM2, for example, is configured by RNN, and includes an input layer, an intermediate layer (a hidden layer), and an output layer. The input layer includes T nodes to which time-series data (Y₁, Y₂, ..., Y_{T}) with a length T is input. Here, Y₁, Y₂, ..., Y_{T}, for example, are values at each time, such as the respiration rate, the heart rate, and the body movement value included in the biological data. The intermediate layer includes a plurality of nodes for executing the arithmetic operation using set parameters (weight and bias) with respect to a numerical value given from the node of the input layer. The node of the output layer outputs a numerical value (Z_{T}) obtained by performing suitable conversion processing with respect to the numerical value given from the node of the intermediate layer. The numerical value Z_{T} output from the output layer is a numerical value indicating the sensitivity information at the time T. By sequentially shifting the time-series data input to the sensitivity information estimation model LM2, the control unit 11 is capable of acquiring the time-series data of the sensitivity information from the sensitivity information estimation model LM2.

Note that, a method for generating the sensitivity information estimation model LM2 will be described in detail in Embodiment 2, and learning is performed with a predetermined learning algorithm by using a data set including the biological data obtained from the biological information estimation model LM1 and the sensitivity information (ground truth data) in training data, and thus, the sensitivity information estimation model LM2 can be generated.

The sensitivity information estimation model LM2 in FIG. 4 includes two intermediate layers, but the number of intermediate layers is not limited to 2, and the intermediate layer can be suitably designed. The sensitivity information estimation model LM2 may be a learning model configured by a long short-term memory (LSTM), convolutional neural networks (CNN), region-based CNN (R-CNN), and the like. The data input to the sensitivity information estimation model LM2 may be prepared in accordance with the neural network configuring the sensitivity information estimation model LM2. For example, in a case where the sensitivity information estimation model LM2 is configured by the CNN or the R-CNN, image data of a graph plotted with time on a horizontal axis and at least one type of the respiration rate, the heart rate, and the body movement value on a vertical axis may be used as the input to the input layer, instead of the time-series data.

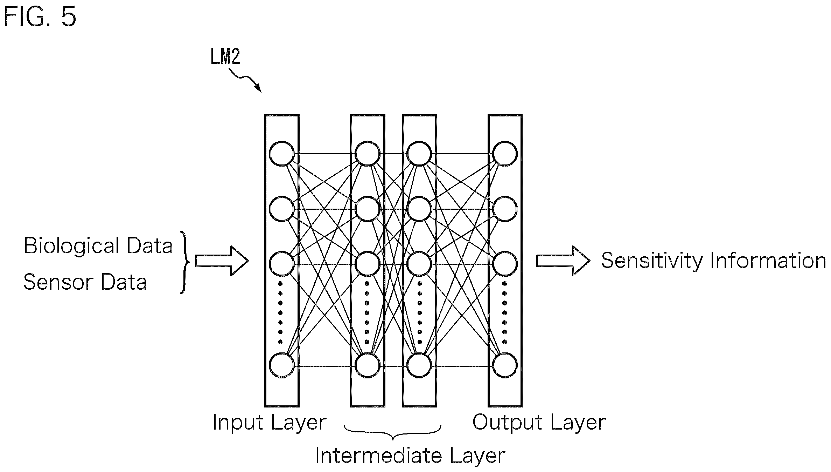

FIG. 5 is a schematic view illustrating a modification example of the sensitivity information estimation model LM2. In a case where the biological data of the user and the sensor data are input, the sensitivity information estimation model LM2 illustrated in FIG. 5 as the modification example is a trained learning model trained to output the sensitivity information of the user. The sensor data includes at least one of the environmental temperature data, the body temperature data of the user, and the blood pressure data of the user, which are obtained by the thermometer S2, the clinical thermometer S3, and the sphygmomanometer S4, in an environment where measurement is performed by using the piezoelectric element S1. In addition, the sensor data may include at least one of the blood oxygen saturation level of the user, the indoor humidity, the illuminance, and the noise level. Note that, the configuration of the sensitivity information estimation model LM2 illustrated in FIG. 5 as the modification example is the same as that illustrated in FIG. 4, and thus, the detailed description thereof will be omitted.

Hereinafter, the operation of the information processing device 1 will be described.

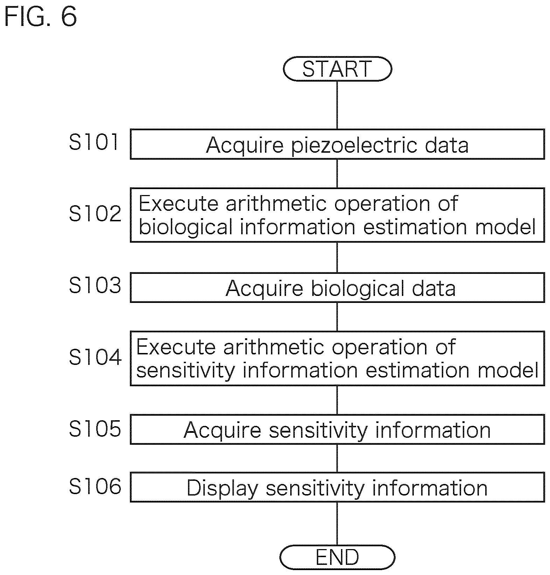

FIG. 6 is a flowchart illustrating the procedure of processing executed by the information processing device 1 during estimation. The user is seated on the chair C in which the piezoelectric element S1 is built in the seat portion, and the piezoelectric element S1 that is built in the chair C is connected to the input unit 13 of the information processing device 1. In a state where the measurement of the piezoelectric element S1 is started, the information processing device 1 executes the following processing. The control unit 11 of the information processing device 1 acquires the piezoelectric data based on the output of the piezoelectric element S1 through the input unit 13 (step S101). In this case, the control unit 11 may acquire the sensor data through the input unit 13.

The control unit 11 inputs the acquired piezoelectric data to the biological information estimation model LM1, and executes the arithmetic operation of the biological information estimation model LM1 (step S102). That is, the control unit 11 inputs each value of the piezoelectric data given as the time-series data to each node of the input layer of the biological information estimation model LM1, executes the arithmetic operation using an activating function including the weight and the bias between the nodes in the intermediate layer, and obtains the final value by converting a value obtained by the intermediate layer using a suitable function. In a case where the biological information estimation model LM1 is trained to output the biological data in accordance with the input of the piezoelectric data and the sensor data, the output of the biological information estimation model LM1 may be obtained by inputting the acquired piezoelectric data and the sensor data.

The control unit 11 repeatedly executes the arithmetic operation of step S102, on the basis of the time-series piezoelectric data acquired in step S101, and thus, acquires the time-series biological data from the output layer of the biological information estimation model LM1 (step S103).

The control unit 11 inputs the acquired biological data to the sensitivity information estimation model LM2, and executes the arithmetic operation of the sensitivity information estimation model LM2 (step S104). That is, the control unit 11 inputs each value of the biological data given as the time-series data to each node of the input layer of the sensitivity information estimation model LM2, executes the arithmetic operation using an activating function including the weight and the bias between the nodes in the intermediate layer, and obtains the final value by converting a value obtained by the intermediate layer using a suitable function.

The control unit 11 sequentially executes the arithmetic operation of step S104, on the basis of the time-series piezoelectric data acquired in step S103, and thus, acquires the time-series sensitivity information from the output layer of the sensitivity information estimation model LM2 (step S105).

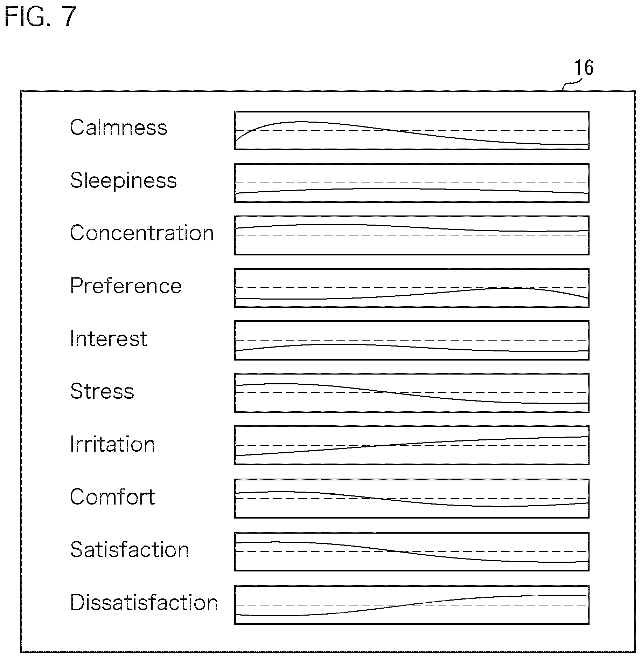

The control unit 11 displays the acquired sensitivity information on the display unit 16 (step S106). FIG. 7 is a schematic view illustrating a display example of the sensitivity information. FIG. 7 illustrates an example in which the calmness, the sleepiness, the concentration, the preference, the interest, the stress, the irritation, the comfort, the satisfaction, and the dissatisfaction are digitized to be displayed as the time-series graph. Alternatively, the control unit 11 may standardize a numerical value indicating the calmness, the sleepiness, the concentration, the preference, the interest, the stress, the irritation, the comfort, the satisfaction, and the dissatisfaction, on the basis of a suitable index, may specify the dominant sensitivity for the user on the basis of the level of the standardized numerical value, and may display information of the specified sensitivity on the display unit 16. In addition, the control unit 11 may notify the sensitivity information to the outer device (for example, a terminal device carried by the user) by the communication unit 14, instead of the configuration in which the sensitivity information is displayed on the display unit 16.

As described above, in Embodiment 1, it is possible to output the sensitivity information of the user, on the basis of the measurement result of the piezoelectric element S1.

### (Embodiment 2)

In Embodiment 2, the method for generating the sensitivity information estimation model LM2 will be described.

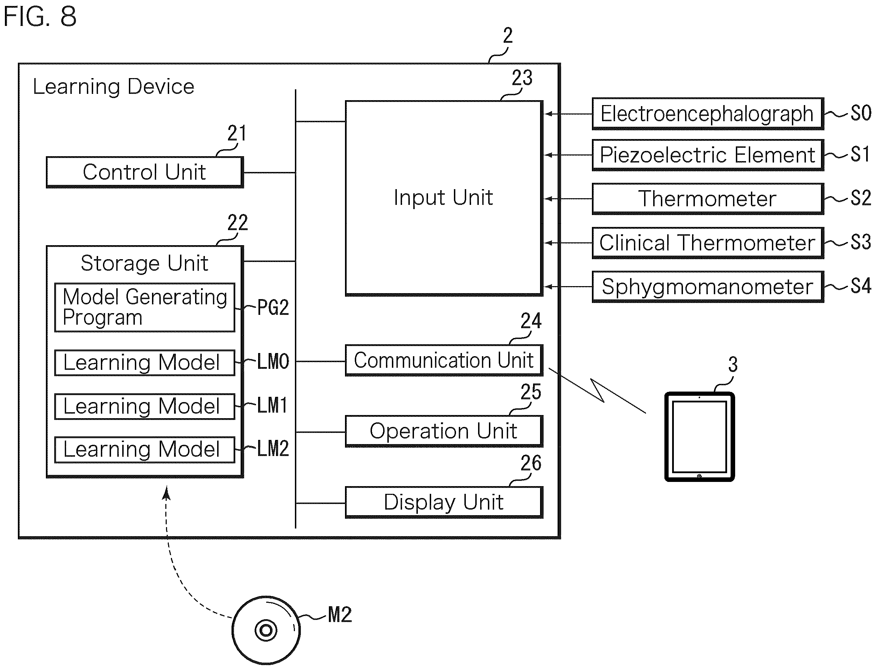

FIG. 8 is a block diagram illustrating the configuration of a learning device according to Embodiment 2. A learning device 2, for example, is a server device connected to the information processing device 1 such that communication is available, and includes a control unit 21, a storage unit 22, an input unit 23, a communication unit 24, an operation unit 25, a display unit 26, and the like. The learning device 2 generates the sensitivity information estimation model LM2, on the basis of the data input from the input unit 23. The trained sensitivity information estimation model LM2 generated by the learning device 2 is downloaded by the information processing device 1, and is installed in the storage unit 12 of the information processing device 1.

The control unit 21, for example, includes a CPU, a ROM, a RAM, and the like. In the ROM of the control unit 21, a control program for controlling the operation of each hardware unit of the learning device 2, and the like are stored. The CPU in the control unit 21 reads in and executes the control program stored in the ROM or various computer programs stored in the storage unit 22, and controls the operation of each hardware unit, and thus, allows the entire device to function as a device attaining a model generating method according to the present invention. In the RAM of the control unit 21, data that is used in the execution of an arithmetic operation is temporarily stored.

The control unit 21 includes the CPU, the ROM, and the RAM, and may be one or a plurality of arithmetic circuits including a GPU, a FPGA, a DSP, a quantum processor, a volatile or non-volatile memory, and the like. In addition, the control unit 21 may have a function such as a clock outputting date and time information, a timer measuring an elapsed time from when a measurement start instruction is given to when a measurement end instruction is given, and a counter counting the number.

The storage unit 22 includes a storage such as a HDD and a SSD. In the storage unit 22, various computer programs executed by the control unit 21 or various data pieces used by the control unit 21 are stored.

The computer program stored in the storage unit 22 includes a model generating program PG2 for executing processing of generating the sensitivity information estimation model LM2 by the following method. The model generating program PG2 may be a single computer program, or may be configured by a plurality of computer programs. In addition, the existing library may be partially used in the model generating program PG2.

The computer program such as the model generating program PG2 stored in the storage unit 22 is provided by a non-temporary recording medium M2 in which the computer program is recorded to be readable. The recording medium M2, for example, is a portable memory such as a CD-ROM, a USB memory, a secure digital (SD) card, a micro SD card, and a compact flash (Registered Trademark). The control unit 21 reads out various computer programs from the recording medium M2 by using a reader that is not illustrated, and stores the various read computer programs in the storage unit 22. In addition, the computer program stored in the storage unit 22 may be provided by communication through the communication unit 24. In this case, the control unit 21 may acquire the computer program through the communication unit 24, and may store the acquired computer program in the storage unit 22.

In a case where electroencephalographic data based on the output of an electroencephalograph S0 is input, the storage unit 22 includes a trained learning model LM0 (hereinafter, also referred to as an electroencephalographic model LM0) trained to output sensitivity information of a subject, a trained learning model LM1 (a biological information estimation model LM1) trained to output biological data of the subject in a case where the signal output from the piezoelectric element S1 is input, and a learning target learning model LM2 (a sensitivity information estimation model LM2) trained to output the sensitivity information of the subject in a case where the biological data of the subject is input. In the storage unit 22, configuration information of a layer of each of the learning models LM0, LM1, and LM2, information of nodes included in each layer, information of weighting and bias between the nodes, and the like are stored as information for defining the learning models LM0, LM1, and LM2. A specific configuration of the learning model LM0 will be described below in detail. The configuration of the learning models LM1 and LM2 is the same as that described in Embodiment 1. Note that, before the learning model LM2 is generated, the initial value is set in parameters (such as the weighting or the bias between the nodes) for characterizing the learning model LM2.

The input unit 23 includes an interface for connecting various sensors. The sensor connected to the input unit 23 includes the electroencephalograph S0, in addition to the piezoelectric element S1, the thermometer S2, the clinical thermometer S3, and the sphygmomanometer S4 described above. The electroencephalograph S0 detects the electroencephalogram of the subject on a time-series basis, and outputs a signal (an electroencephalographic signal) indicating a detection result to the learning device 2. The control unit 21 of the learning device 2 acquires time-series data (hereinafter, referred to as the electroencephalographic data) based on the output of the piezoelectric element S1 through the input unit 23. The electroencephalographic data acquired by the control unit 21 may be a raw signal obtained from the electroencephalograph S0, or may be data obtained by processing the electroencephalographic signal. For example, the control unit 21 may perform processing of resolving the electroencephalographic signal input through the input unit 23 to α waves, β waves, γ waves, δ waves, and θ waves to use such waveform data as the electroencephalographic data. In addition, the control unit 21 may perform processing of extracting some feature amounts from the electroencephalographic signal input through the input unit 23 to use time-series data of the obtained feature amounts as the electroencephalographic data. Hereinafter, the raw signal (the electroencephalographic signal) obtained from the electroencephalograph S0 will be referred to as the electroencephalographic data without being distinguished from the data obtained after the processing.

Further, various sensors such as a sensor measuring the blood oxygen saturation level of the subject, a sensor measuring the indoor humidity, a sensor measuring an illuminance, and a sensor measuring a noise level may be connected to the input unit 23.

The communication unit 24 includes a communication interface for transmitting and receiving various data pieces. The communication interface of the communication unit 24, for example, is a communication interface based on communication standard of a local area network (LAN) used in WiFi (Registered Trademark) or Ethernet (Registered Trademark). In a case where data to be transmitted is input from the control unit 21, the communication unit 24 transmits the data to be transmitted to a designated destination. In addition, in a case where data transmitted from an outer device is received, the communication unit 24 outputs the received data to the control unit 21. An example of the outer device connected to the communication unit 24 such that communication is available is a tablet terminal 3 operated by the subject. Another example of the outer device connected to the communication unit 24 is the information processing device 1 described in Embodiment 1.

The operation unit 25 includes an operation device such as a touch panel, a keyboard, and a switch, and receives various operations and settings of an administrator or the subject. The control unit 21 performs suitable control on the basis of various operation information pieces given from the operation unit 25, and as necessary, stores setting information in the storage unit 22.

The display unit 26 includes a display device such as a liquid crystal monitor or an organic electro-luminescence (EL), and displays information to be notified to the administrator or the subject, in accordance with an instruction from the control unit 21.

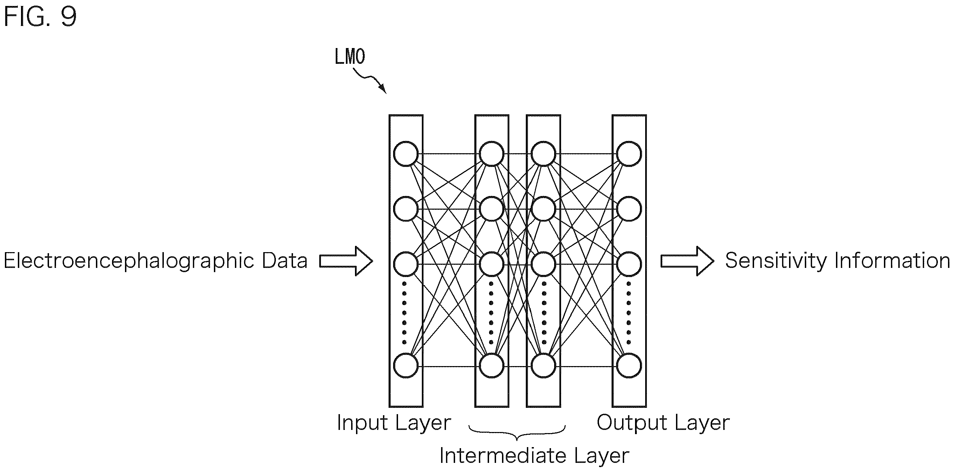

FIG. 9 is a schematic view illustrating a configuration example of the electroencephalographic model LM0. In a case where the electroencephalographic data of the subject is input, the electroencephalographic model LM0 in Embodiment 2 is a trained learning model trained to output the sensitivity information of the subject. The electroencephalographic data input to the electroencephalographic model LM0 is time-series electroencephalographic data based on the output of the electroencephalograph S0. In addition, the sensitivity information output from the sensitivity information estimation model LM2 is time-series data obtained by digitizing at least one of the calmness, the sleepiness, the concentration, the preference, the interest, the stress, the irritation, the comfort, the satisfaction, and the dissatisfaction of the subject.

The electroencephalographic model LM0, for example, is configured by RNN, and includes an input layer, an intermediate layer (a hidden layer), and an output layer. The input layer includes T nodes to which time-series data (B₁, B₂, ..., B_{T}) with a length T is input. Here, B₁, B₂, ... , B_{T}, for example, are values at each time included in the electroencephalographic data. The intermediate layer includes a plurality of nodes for executing the arithmetic operation using set parameters (weight and bias) with respect to a numerical value given from the node of the input layer. The node of the output layer outputs a numerical value (Z_{T}) obtained by performing suitable conversion processing with respect to the numerical value given from the node of the intermediate layer. The numerical value Z_{T} output from the output layer is a numerical value indicating the sensitivity information at the time T. By sequentially shifting the time-series data input to the electroencephalographic model LM0, the control unit 21 is capable of acquiring the time-series data of the sensitivity information from the electroencephalographic model LM0.

As a method for generating the electroencephalographic model LM0, the existing method for generating an electroencephalographic model may be used. That is, learning is performed with a predetermined learning algorithm by using a data set including the electroencephalographic data obtained by the electroencephalograph S0, and the sensitivity information (ground truth data) in training data, and thus, the electroencephalographic model LM0 can be generated. Note that, the sensitivity information (the ground truth data) for the learning may be given from the subject oneself by using the tablet terminal 3.

The electroencephalographic model LM0 in FIG. 9 includes two intermediate layers, but the number of intermediate layers is not limited to 2, and the intermediate layer can be suitably designed. The electroencephalographic model LM0 may be a learning model configured by a LSTM, CNN, R-CNN, and the like. The data input to the electroencephalographic model LM0 may be prepared in accordance with the neural network configuring the electroencephalographic model LM0. For example, in a case where the electroencephalographic model LM0 is configured by the CNN or the R-CNN, image data such as a graph plotted with time on a horizontal axis and at least one type of wave height on a vertical axis, and a contour map plotted by allowing feature amounts obtained at each time to correspond to electroencephalographic measurement sites may be used as the input to the input layer, instead of the time-series data.

Hereinafter, the operation of the learning device 2 will be described.

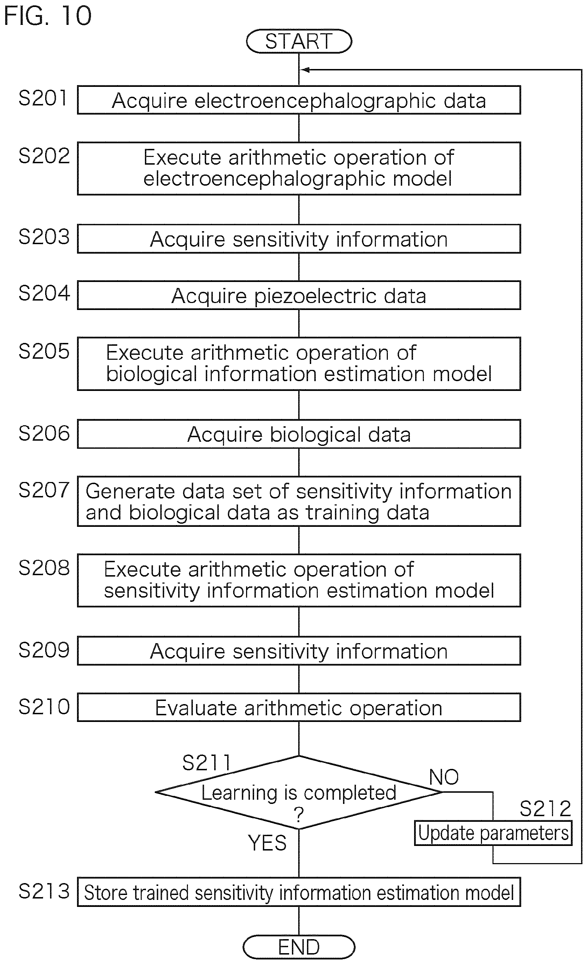

FIG. 10 is a flowchart illustrating a generating procedure of the training data. The subject is seated on the chair C in which the piezoelectric element S1 is built in the seat portion, and the electroencephalograph S0 is mounted on the head of the subject. The electroencephalograph S0 and the piezoelectric element S1 are connected to the input unit 23 of the learning device 2, and in a state where the measurement of the electroencephalograph S0 and the piezoelectric element S1 is started, the learning device 2 executes the following processing.

The control unit 21 of the learning device 2 includes the input unit 23, and acquires the electroencephalographic data based on the output of the electroencephalograph S0 (step S201).

The control unit 21 inputs the acquired electroencephalographic data to the electroencephalographic model LM0, and executes the arithmetic operation of the electroencephalographic model LM0 (step S202). That is, the control unit 21 inputs each value of the electroencephalographic data given as the time-series data to each node of the input layer of the electroencephalographic model LM0, executes the arithmetic operation using an activating function including the weight and the bias between the nodes in the intermediate layer, and obtains the final value by converting a value obtained by the intermediate layer using a suitable function.

The control unit 21 repeatedly executes the arithmetic operation of step S202, on the basis of the time-series electroencephalographic data acquired in step S201, and thus, acquires the time-series sensitivity information from the output layer of the electroencephalographic model LM0 (step S203).

The control unit 21 acquires the piezoelectric data based on the output of the piezoelectric element S1 through the input unit 23 (step S204). In this case, the control unit 21 may acquire the sensor data through the input unit 23.

The control unit 21 inputs the acquired piezoelectric data to the biological information estimation model LM1, and executes the arithmetic operation of the biological information estimation model LM1 (step S205). That is, the control unit 21 inputs each value of the piezoelectric data given as the time-series data to each node of the input layer of the biological information estimation model LM1, executes the arithmetic operation using an activating function including the weight and the bias between the nodes in the intermediate layer, and obtains the final value by converting a value obtained by the intermediate layer using a suitable function. In a case where the biological information estimation model LM1 is trained to output the biological data in accordance with the input of the piezoelectric data and the sensor data, the output of the biological information estimation model LM1 may be obtained by inputting the acquired piezoelectric data and the sensor data.

The control unit 21 repeatedly executes the arithmetic operation of step S205, on the basis of the time-series piezoelectric data acquired in step S204, and thus, acquires the time-series biological data from the output layer of the biological information estimation model LM1 (step S206).

The control unit 21 generates a data set of the time-series sensitivity information obtained in step S203 and the time-series biological data obtained in step S206 as the training data (step S207).

The control unit 21 inputs the acquired biological data to the sensitivity information estimation model LM2, and executes the arithmetic operation of the sensitivity information estimation model LM2 (step S208). In the initial stage before the learning is started, the initial value is set in the parameters (the weight and the bias) of the sensitivity information estimation model LM2. The control unit 21 inputs each value of the biological data given as the time-series data to each node of the input layer of the sensitivity information estimation model LM2, executes the arithmetic operation using an activating function including the weight and the bias between the nodes in the intermediate layer, and obtains the final value by converting a value obtained by the intermediate layer using a suitable function.

The control unit 21 repeatedly executes the arithmetic operation acquired in step S208, on the basis of the time-series biological data acquired in step S206, and thus, acquires the time-series sensitivity information from the output layer of the sensitivity information estimation model LM2 (step S209).

For convenience of description, in the flowchart of FIG. 10, a procedure is described in which the arithmetic operation of the electroencephalographic model LM0, the arithmetic operation of the biological information estimation model LM1, and the arithmetic operation of the sensitivity information estimation model LM2 are executed in this order, but the arithmetic operations are simultaneously carried out in parallel.

The control unit 21 evaluates the arithmetic operation result by using the sensitivity information included in the training data generated in step S207 and the sensitivity information acquired in step S209 (step S210), and determines whether the learning is completed (step S211). That is, the control unit 21 may evaluate the sensitivity information of the sensitivity information estimation model LM2 (the sensitivity information estimated on the basis of the piezoelectric data) by using the sensitivity information (the sensitivity information obtained from the electroencephalographic data) included in the training data as the ground truth data. Specifically, the control unit 21 is capable of evaluating the arithmetic operation result by using an error function (also referred to as an objective function, a loss function, and a cost function) based on the ground truth data and the arithmetic operation result obtained in step S208. In a case where the error function is a threshold value or less (or a threshold value or more) in a process where the error function is optimized (minimized or maximized) by a gradient descent method such as a steepest descent method, the control unit 21 determines that the learning is completed. Note that, in order to avoid the problem of over-learning, a method such as cross-validation and early stopping may be incorporated to end the learning at a suitable timing.

In a case where it is determined that the learning is not completed (S211: NO), the control unit 21 updates the parameters (the weight and the bias between the nodes) of the sensitivity information estimation model LM2 (step S212), allows the processing to return to step S201, and continues the learning. The control unit 21 is capable of proceeding with the learning toward the input layer from the output layer of the sensitivity information estimation model LM2 by using an error back propagation algorithm for sequentially updating the weight and the bias between the nodes.

In a case where it is determined that the learning is completed (S211: YES), the control unit 21 stores the sensitivity information estimation model LM2 in the storage unit 22 as a trained learning model (step S213), the processing of this flowchart is ended. The generated trained sensitivity information estimation model LM2 is transmitted to the information processing device 1, and is stored in the storage unit 12 of the information processing device 1.

Note that, in this embodiment, the sensitivity information obtained from the electroencephalographic model LM0 is used as the ground truth data, but sensitivity information (ground truth data) given by the subject oneself using the tablet terminal 3 may be used together. For example, in the initial stage after the learning is started, the learning is performed by using the sensitivity information given by the subject oneself using the tablet terminal 3 in the ground truth data, and in a stage where the learning has proceeded, the learning may be continued with the sensitivity information obtained from the electroencephalographic model LM0 as the ground truth data.

As described above, in Embodiment 2, the learning is performed by using the sensitivity information of the subject that is estimated using the electroencephalographic data obtained by the electroencephalograph S0 in the ground truth data, and thus, it is possible to generate the sensitivity information estimation model LM2.

### (Embodiment 3)

In Embodiment 3, relearning processing of the sensitivity information estimation model LM2 will be described.

The learning device 2 is capable of estimating the sensitivity of the user by using the trained sensitivity information estimation model LM2. An estimation procedure is the same as that in Embodiment 1. The learning device 2 specifies the dominant sensitivity for the user on the basis of the sensitivity information acquired from the sensitivity information estimation model LM2, and displays information of the specified sensitivity on the display unit 26 such that the user checks whether the sensitivity information is correct.

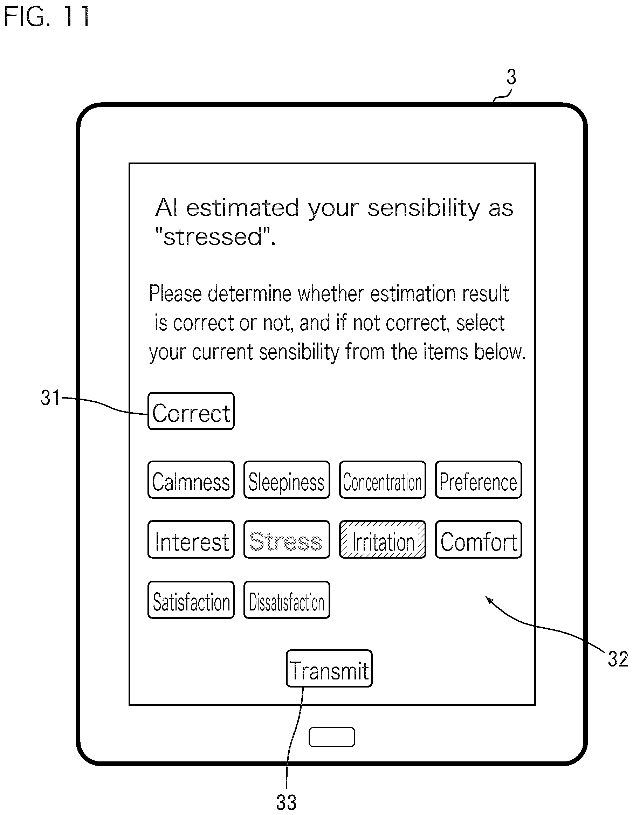

FIG. 11 is a schematic view illustrating an example of a confirmation window. FIG. 11 illustrates the confirmation window of the tablet terminal 3 operated by the user. On the confirmation window, a selection button 31 selected in a case where the estimation result is correct, an item button group 32 selected in a case where the estimation result is not correct, and a transmission button 33 operated when transmitting a selection result are arranged together with an estimation result of the sensitivity information estimation model LM2, and a message for checking with the user whether the estimation result is correct. In a case where the estimation result of the sensitivity information estimation model LM2 is correct, the user selects the selection button 31, and presses the transmission button 33, and thus, performs a response to the information processing device 1. In addition, in a case where the estimation result of the sensitivity information estimation model LM2 is not correct, the user selects any button of the item button group 32, and presses the transmission button 33, and thus, performs the response to the information processing device 1. The selection result is transmitted to the learning device 2 from the tablet terminal 3.

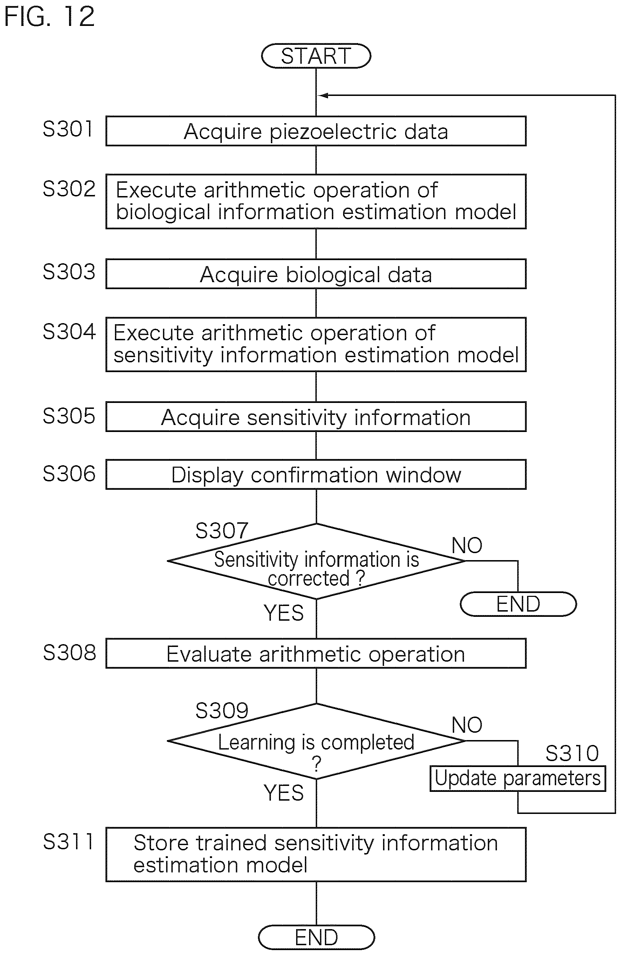

FIG. 12 is a flowchart illustrating a relearning procedure executed by the learning device 2. The control unit 21 of the learning device 2 acquires the sensitivity information of the user by executing the same procedure as that in Embodiment 1 (steps S301 to 305). The control unit 21 displays the confirmation window for checking with the user whether the estimation result is correct on the display unit 26, on the basis of the acquired sensitivity information (step S306).

The control unit 21 determines whether the sensitivity information is corrected, on the basis of the response (the selection result) transmitted from the tablet terminal 3 (step S307). In a case where the sensitivity information is not corrected (S307: NO), the control unit 21 ends the processing of this flowchart.

In a case where it is determined that the sensitivity information is corrected (S307: YES), the control unit 21 evaluates the arithmetic operation result of the sensitivity information estimation model LM2 by using the sensitivity information corrected by the user, and the sensitivity information obtained in S305 (step S308), and determines whether the learning is completed (step S309).

In a case where it is determined that the learning is not completed (S309: NO), the control unit 21 updates the parameters (the weight and the bias between the nodes) of the sensitivity information estimation model LM2 (step S310), allows the processing to return to step S301, and continues the learning by evaluating the sensitivity information of the subject that is estimated on the basis of the output of the piezoelectric element S1 using the sensitivity information corrected by the subject as the ground truth data. The control unit 21 is capable of proceeding with the learning toward the input layer from the output layer of the sensitivity information estimation model LM2 by using an error back propagation algorithm for sequentially updating the weight and the bias between the nodes.

In a case where it is determined that the learning is completed (S309: YES), the control unit 21 stores the sensitivity information estimation model LM2 in the storage unit 22 as a retrained learning model (step S311), and ends the processing of this flowchart. The generated retrained sensitivity information estimation model LM2 is transmitted to the information processing device 1, and is stored in the storage unit 12 of the information processing device 1.

As described above, in Embodiment 3, the sensitivity information estimation model LM2 can be retrained by receiving the correction of the sensitivity information from the user, and thus, it is possible to generate the sensitivity information estimation model LM2 with a higher estimation accuracy.

### (Embodiment 4)

In Embodiment 4, a configuration will be described in which the sensitivity information of the user is estimated in various environments such as an office space, a home, and a commuting vehicle to evaluate each environment.

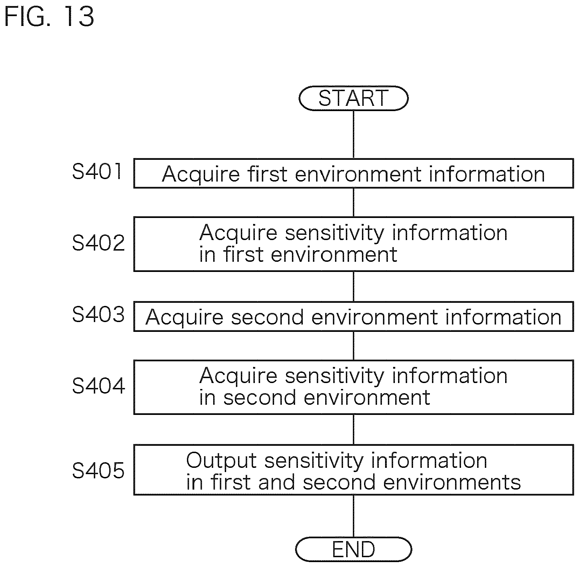

FIG. 13 is a flowchart illustrating the procedure of processing executed by the information processing device 1 according to Embodiment 4. The control unit 11 of the information processing device 1 acquires first environment information through the operation unit 15 (step S401). The environmental information, for example, indicates information of a location where the user exists. The environmental information may further include information such as the weather, the ambient temperature, the humidity, an illuminance, a noise level, and a time zone.

The control unit 11 acquires the sensitivity information of the user in a first environment (step S402). That is, the control unit 11 executes the procedure illustrated in the flowchart of FIG. 6 in the first environment, and thus, is capable of acquiring the sensitivity information of the user from the sensitivity information estimation model LM2. The acquired sensitivity information is stored in the storage unit 12.

Next, the control unit 11 acquires second environment information different from the first environment information through the operation unit 15 (step S403). Here, in a case where the user moves to a second environment from the first environment, environmental information after the movement may be acquired.

The control unit 11 acquires the sensitivity information of the user in the second environment (step S404). That is, the control unit 11 executes the procedure illustrated in the flowchart of FIG. 6 in the second environment, and thus, is capable of acquiring the sensitivity information of the user from the sensitivity information estimation model LM2. The acquired sensitivity information is stored in the storage unit 12.

The control unit 11 outputs the first environment information and the sensitivity information obtained in the first environment in association with each other, and outputs the second environment information and the sensitivity information obtained in the second environment in association with each other (step S405). The control unit 11 may output such information by displaying the information on the display unit 16.

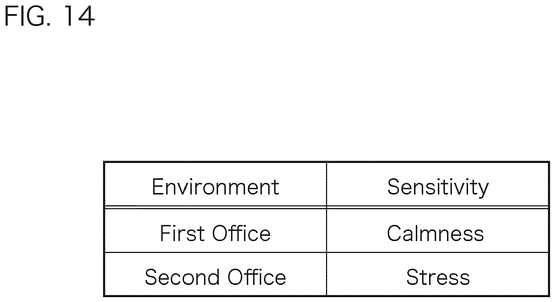

FIG. 14 is a schematic view illustrating an output example in Embodiment 4. The example of FIG. 14 illustrates a change in the sensitivity in a case where the environment of the user is changed to a second office (the second environment) from a first office (the first environment). Since the example of FIG. 14 illustrates that the sensitivity of the user is changed to a tense state from a calm state in accordance with the movement to the second office from the first office, the information processing device 1 may propose the environmental improvement of the second office.

As described above, in Embodiment 4, it is possible to provide a change in the sensitivity of the user according to an environmental change as information.

Note that, the information processing device 1 according to Embodiment 4 is configured to display a change in the sensitivity information of the user according to the environmental change on the display unit 16, but various devices such as an air conditioner, a blind device, an illumination device, and an acoustic device may be controlled in accordance with the specified sensitivity information of the user. The control of such devices may be performed by transmitting a control signal to each of the devices from the communication unit 14 of the information processing device 1. For example, in a case where it is estimated that the sleepiness of the user increases, the information processing device 1 may transmit a control signal for instructing to decrease the room temperature to the air conditioner, or may transmit a control signal for instructing to increase the illuminance to the illumination device. In addition, in a case where it is estimated that the concentration of the user decreases, the information processing device 1 may transmit a control signal for instructing to lower the blind to the blind device. Further, in a case where it is estimated that the stress or the irritation of the user increases, the information processing device 1 may transmit the control signal to the acoustic device such that music for relieving the stress or the irritation is replayed. As described above, the information processing device 1 is capable of controlling various devices in accordance with the sensitivity information of the user.

The information processing device 1 may specify sensitivity information for each of a plurality of users in the same environment, and may derive the average sensitivity information of the plurality of users. In addition, the information processing device 1 may notify the derived average sensitivity information to a terminal of a group leader or an administrator.

The information processing device 1 may acquire work performance through the communication unit 14, and may record and output the acquired work performance and the sensitivity information of the user when performing work in association with each other.

In a case where a web conference is performed by using a web conferencing app or in a case where a lecture or a seminar is performed by using a presentation app, the information processing device 1 may specify sensitivity information of a participant, and may display the specified sensitivity information on a screen of the app. When the web conference is performed, the information processing device 1 provides sensitivity information of a conference opponent to a speaker, and thus, is capable of applying a more trouble-free conference space. In addition, when the presentation is performed, the information processing device 1 provides sensitivity information of the audience to a presenter, and thus, is capable of contribution to the quality improvement of the presentation.

In a case where a class is performed at a school, a tutoring school, or the like, the information processing device 1 may acquire sensitivity information of a student, and may notify the acquired sensitivity information to a terminal of a teacher or a lector. The information processing device 1 acquires the sensitivity information of the student during the class, and provides the acquired sensitivity information to the teacher or the lector, and thus, is capable of contributing to the quality improvement of the class.

The information processing device 1 may acquire the sensitivity information of not only the user in the office but also a manipulator of an automobile, an electric train, an airplane, or the like. In addition, the information processing device 1 may acquire sensitivity information of a player of a game, e-sports, or the like. Further, the information processing device 1 may acquire sensitivity information of a viewer of sports viewing, game viewing, a theatrical play, a play, a movie, or the like.

### (Embodiment 5)

In Embodiment 5, a configuration will be described in which the sensitivity information of the user is estimated in a state where various offerings such as perfume and aroma are provided to evaluate each of the offerings.

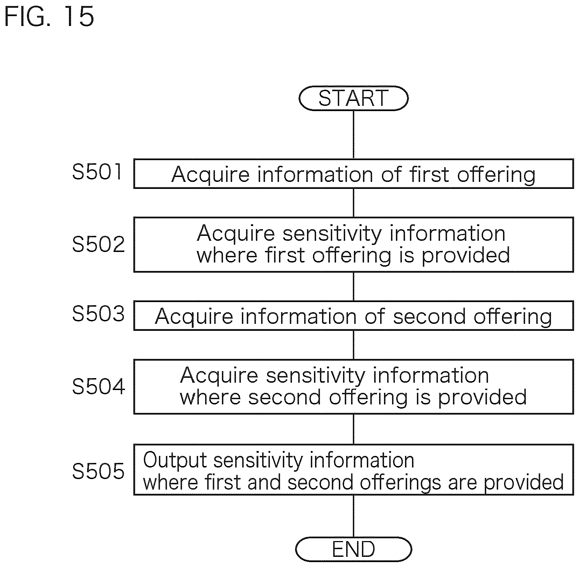

FIG. 15 is a flowchart illustrating the procedure of processing executed by the information processing device 1 according to Embodiment 5. The control unit 11 of the information processing device 1 acquires information of a first offering through the operation unit 15 (step S501). The offering includes an intangible article such as perfume and aroma.

In a state where the first offering is provided, the control unit 11 acquires the sensitivity information of the user (step S502). That is, in a state where the first offering is provided, the control unit 11 executes the procedure illustrated in the flowchart of FIG. 6, and thus, is capable of acquiring the sensitivity information of the user from the sensitivity information estimation model LM2. The acquired sensitivity information is stored in the storage unit 12.

Next, the control unit 11 acquires information of a second offering different from the first offering through the operation unit 15 (step S503). Here, in a case where the offering provided to the user is changed to the second offering from the first offering, the information of the offering after the change may be acquired.

In a state where the second offering is provided, the control unit 11 acquires the sensitivity information of the user (step S504). That is, in a state where the second offering is provided, the control unit 11 executes the procedure illustrated in the flowchart of FIG. 6, and thus, is capable of acquiring the sensitivity information of the user from the sensitivity information estimation model LM2. The acquired sensitivity information is stored in the storage unit 12.

The control unit 11 outputs the information of the first offering and the sensitivity information obtained in a state where the first offering is provided in association with each other, and outputs the information of the second offering and the sensitivity information in a state where the second offering is provided in association with each other (step S505). The control unit 11 may output such information by displaying the information on the display unit 16.

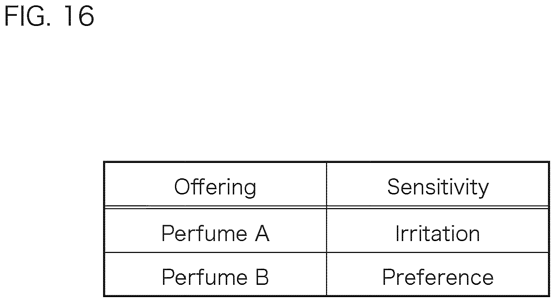

FIG. 16 is a schematic view illustrating an output example in Embodiment 5. The example of FIG. 16 illustrates a change in the sensitivity in a case where the offering provided to the user is changed to perfume B (the second offering) from perfume A (the first offering). The example of FIG. 16 illustrates that the sensitivity of the user is changed to the preference from the irritation in accordance with a change to the perfume B from the perfume A, and thus, the information processing device 1 is capable of evaluating that the perfume B is more preferable for the user.

As described above, in Embodiment 5, it is possible to provide a change in the sensitivity of the user according to a change in the offering as information.

The offering may be not only the perfume or the aroma, but also a cosmetic product or a medicinal product. In addition, the information processing device 1 may acquire the sensitivity information of the user based on the sense of taste, before and after the food, the beverage, or the like is provided. Further, the information processing device 1 may acquire the sensitivity information of the user based on the sense of vision, before and after a painting, a picture, or the like is provided. Further, the information processing device 1 may acquire the sensitivity information of the user based on the sense of touch, before and after a product with features in a tactual sense or a texture is provided. Further, the information processing device 1 may acquire the sensitivity information of the user based on the sense of hearing, before and after the music is provided. As described above, the information processing device 1 according to this embodiment is capable of acquiring the sensitivity information of the user when not only a tangible article or an intangible article but also various offerings are provided.

Further, in a case where the sensitivity when the offering is provided is received as a response for a questionnaire, the information processing device 1 may acquire the sensitivity information of the user that is specified by the sensitivity information estimation model LM2 as the response, instead of the input by the user.

### (Embodiment 6)

In Embodiment 6, a configuration will be described in which the sensitivity information estimation model LM2 is selected in accordance with the attribute of the user.

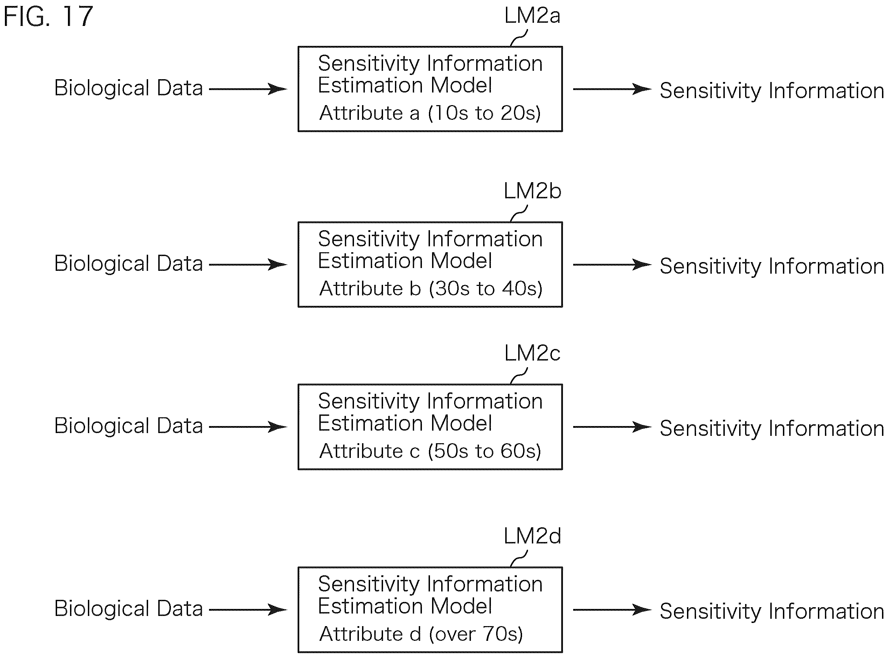

FIG. 17 is an explanatory diagram illustrating a learning model prepared in Embodiment 6. In Embodiment 6, the sensitivity information estimation model LM2 is prepared in accordance with the attribute of the user. Here, the attribute of the user is information for characterizing the user such as an age, a gender, a resident area, an academic record, a work record, and a family configuration. The learning device 2 performs the measurement of the electroencephalograph S0 or the measurement of the piezoelectric element S1 with respect to various subjects with different attribute, thereby generating the sensitivity information estimation model LM2 for each attribute. The sensitivity information estimation model LM2 for each attribute that is generated by the learning device 2 is transmitted to the information processing device 1, and is stored in the storage unit 12 of the information processing device 1.

The example of FIG. 17 illustrates that a sensitivity information estimation model LM2a for attribute a (the teens to the twenties), a sensitivity information estimation model LM2b for attribute b (the thirties to the forties), a sensitivity information estimation model LM2c for attribute c (the fifties to the sixties), and a sensitivity information estimation model LM2d for attribute d (the seventies or more) are prepared as the sensitivity information estimation model LM2 for each attribute.

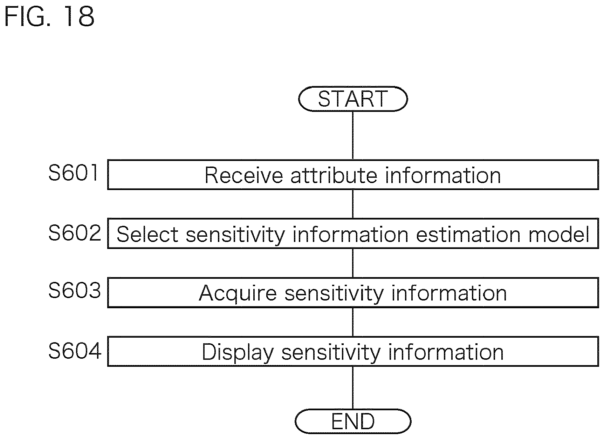

FIG. 18 is a flowchart illustrating the procedure of processing executed by the information processing device 1 according to Embodiment 6. The control unit 11 of the information processing device 1 receives attribute information of the user through the operation unit 15 (step S601). The control unit 11 selects the corresponding sensitivity information estimation model LM2 on the basis of the received attribute information (step S602). In a case where the attribute a (the teens to the twenties) is received as the attribute information, the control unit 11 may select the sensitivity information estimation model LM2a stored in the storage unit 12. The same applies to a case where other attribute information is received.

The control unit 11 acquires the sensitivity information of the user by using the selected sensitivity information estimation model LM2 (step S603). That is, the control unit 11 executes the procedure illustrated in the flowchart of FIG. 6, and thus, may acquire the sensitivity information of the user from the selected sensitivity information estimation model LM2. The control unit 11 displays the acquired sensitivity information of the user on the display unit 16 (step S604).

As described above, in Embodiment 6, the sensitivity of the user is estimated by using the sensitivity information estimation model LM2 selected in accordance with the attribute information of the user, and thus, it is possible to improve the estimation accuracy.

The embodiments disclosed herein are considered illustrative in all respects but not restrictive. The scope of the present invention is indicated by the claims but not the meaning described above, and is intended to include all modifications within the meaning and the scope equivalent to the claims.

### [Description of Reference Numerals]

- 1: Information processing device
- 2: Learning device
- 11: Control unit
- 12: Storage unit
- 13: Input unit
- 14: Communication unit
- 15: Operation unit
- 16: Display unit
- 21: Control unit
- 22: Storage unit
- 23: Input unit
- 24: Communication unit
- 25: Operation unit
- 26: Display unit
- LM0, LM1, LM2: Learning model
- PG1: Estimation processing program
- PG2: Model generating program
- S0: Electroencephalograph
- S1: Piezoelectric element
- S2: Thermometer
- S3: Clinical thermometer
- S4: Sphygmomanometer

## Claims

1. A model generating method causing a computer to execute processing of:
acquiring electroencephalographic data of subjects based on outputs of an electroencephalograph;
acquiring sensitivity information of the subjects by inputting the acquired electroencephalographic data to a first learning model trained to output the sensitivity information in accordance with input of the electroencephalographic data;
acquiring biological data of the subjects based on outputs of a piezoelectric element; and
generating a second learning model for outputting the sensitivity information of a person being tested by using a data set including the acquired biological data and the sensitivity information of the subjects acquired from the first learning model as training data when the biological data of the person measured by the piezoelectric element is input.

2. The model generating method according to claim 1,
wherein the training data further includes sensor data obtained by other sensors, and
when the biological data and the sensor data are input, the second learning model is trained to output the sensitivity information.

3. The model generating method according to claim 1 or 2,
wherein the biological data is at least one time-series data piece of a respiration rate, a heart rate, and a body movement value of the subjects.

4. The model generating method according to claim 2,
wherein the sensor data is at least one time-series data piece of an environmental temperature, and a body temperature and a blood-pressure value of the subjects.

5. The model generating method according to any one of claims 1 to 4,
wherein the sensitivity information includes at least one of calmness, sleepiness, concentration, preference, interest, stress, irritation, comfort, satisfaction, and dissatisfaction of the subjects.

6. The model generating method according to any one of claims 1 to 5,
wherein the piezoelectric element is a line-shaped or film-shaped piezoelectric element containing a non-pyroelectric organic piezoelectric material.

7. The model generating method according to any one of claims 1 to 6,
wherein the biological data is acquired from the piezoelectric element arranged in a state of not being in contact with skins of the subjects while acquiring the electroencephalographic data from the electroencephalograph mounted on heads of the subjects.

8. A computer program causing a computer to execute processing of:
acquiring biological data of a user based on an output of a non-pyroelectric piezoelectric element arranged in a state of not being in contact with a skin of the user;
acquiring sensitivity information of the user by inputting the acquired biological data of the user to a learning model trained to output the sensitivity information in accordance with input of the biological data; and
outputting information based on the acquired sensitivity information.

9. The computer program according to claim 8,
wherein the learning model is trained by using a data set that includes sensitivity information of subjects estimated by using another learning model based on electroencephalographic data of the subjects and includes biological data of the subjects based on outputs of the piezoelectric element as training data.

10. The computer program according to claim 9,
wherein the learning model is trained by using the data set further including sensor data obtained by other sensors as the training data.

11. The computer program according to any one of claims 8 to 10,
wherein the biological data is at least one time-series data piece of a respiration rate, a heart rate, and a body movement value of the user.

12. The computer program according to claim 10,
wherein the sensor data is at least one time-series data piece of an environmental temperature, and a body temperature and a blood-pressure value of the user.

13. The computer program according to any one of claims 8 to 12 for causing the computer to further execute processing of:
acquiring attribute information of the user;
selecting one learning model from a plurality of learning models prepared in accordance with attribute of the user, based on the acquired attribute information of the user; and
acquiring the sensitivity information of the user by inputting the biological data of the user to the selected one learning model.

14. The computer program according to any one of claims 8 to 13 for causing the computer to further execute processing of:
receiving correction of the sensitivity information from the user; and
relearning the learning model by using training data including the corrected sensitivity information and the biological data measured for the user as a data set.

15. The computer program according to any one of claims 8 to 14 for causing the computer to further execute processing of:
acquiring first sensitivity information by inputting first biological data of the user measured by the piezoelectric element in a first environment to the learning model;
acquiring second sensitivity information by inputting second biological data of the user measured by the piezoelectric element in a second environment to the learning model; and
outputting information of the first environment and the first sensitivity information acquired from the learning model in association with each other, and outputting information of the second environment and the second sensitivity information acquired from the learning model in association with each other.

16. The computer program according to any one of claims 8 to 14 for causing the computer to further execute processing of:
acquiring first sensitivity information by inputting first biological data of the user measured by the piezoelectric element in a situation in which a first offering is provided to the user to the learning model;
acquiring second sensitivity information by inputting second biological data of the user measured by the piezoelectric element in a situation in which a second offering is provided to the user to the learning model; and
outputting information of the first offering and the first sensitivity information acquired from the learning model in association with each other, and outputting information of the second offering and the second sensitivity information acquired from the learning model in association with each other.

17. An information processing device, comprising:
a biological data acquisition unit acquiring biological data of a user based on an output of a non-pyroelectric piezoelectric element arranged in a state of not being in contact with a skin of the user;
a sensitivity information acquisition unit acquiring sensitivity information of the user by inputting the acquired biological data of the user to a learning model trained to output the sensitivity information in accordance with input of the biological data; and
an output unit outputting information based on the acquired sensitivity information.

18. An information processing system, comprising:
a non-pyroelectric piezoelectric element arranged in a state of not being in contact with a skin of a user; and
an information processing device that includes:
a biological data acquisition unit acquiring biological data of the user based on an output of the piezoelectric element;
a sensitivity information acquisition unit acquiring sensitivity information of the user by inputting the acquired biological data of the user to a learning model trained to output the sensitivity information in accordance with input of the biological data; and
an output unit outputting information based on the acquired sensitivity information.

19. An information processing method causing a computer to execute processing of:
acquiring biological data of a user based on an output of a non-pyroelectric piezoelectric element arranged in a state of not being in contact with a skin of the user;
acquiring sensitivity information of the user by inputting the acquired biological data of the user to a learning model trained to output the sensitivity information in accordance with input of the biological data; and
outputting information based on the acquired sensitivity information.

20. A training data generating method causing a computer to execute processing of:
acquiring electroencephalographic data of subjects based on an output of an electroencephalograph;
acquiring sensitivity information of the subjects by inputting the acquired electroencephalographic data to a first learning model trained to output the sensitivity information in accordance with input of the electroencephalographic data;
acquiring biological data of the subjects based on output of the non-pyroelectric piezoelectric element arranged in a state of not being in contact with a skin of the subjects; and
generating a data set including the acquired sensitivity information and the acquired biological data as training data used for learning a second learning model trained to output the sensitivity information of the user when the biological data of the user is input.
